# EUROPEAN PATENT APPLICATION

(11) **EP 4 768 056 A1**
(43) Date of publication of application: **01.07.2026**
(21) Application number: 24872747.1
(22) Date of filing: 27.08.2024
(51) Int. Cl.: A61M 5/168, A61M 5/142

(54) **DRUG INJECTION DETECTION UNIT, DRUG INJECTION DEVICE COMPRISING SAME, AND METHOD FOR OPERATING DRUG INJECTION DEVICE**

(30) Priority: 26.09.2023 KR 20230129083
(71) Applicant: Caremedi Co., Ltd., Seoul 07207 (KR)
(72) Inventor: SHIN, Woonsup, Seoul 04065 (KR); ZHU, Enhua, Seoul 04109 (KR); LEE, Junghyun, Seoul 03916 (KR); KWON, Junmo, Yangju-si, Gyeonggi-do 11456 (KR); KIM, Kyunam, Yongin-si, Gyeonggi-do 17161 (KR)
(74) Representative: Manitz Finsterwald Patent- und Rechtsanwaltspartnerschaft mbB
(86) International application number: PCT/KR2024/012750
(87) International publication number: WO 2025/071039

(57) **Abstract**

The drug injection device according to the present invention comprises: a drug storage unit the thickness of which varies according to the amount of drug to be injected; a pump assembly which is connected to the drug storage unit, and draws in and discharges the drug stored in the drug storage unit; and a drug injection detection unit which detects a change in the thickness of the drug storage unit and controls the operation of the drug injection device according to the change in the amount of the drug stored.

## Description

### BACKGROUND

### [Technical Field]

The present invention relates to a drug injection detection unit, a drug injection device comprising same, and a method for operating the drug injection device.

### [Background Art]

Drugs may be administered into a body in various manners such as oral administration, subcutaneous administration, and intravenous administration depending on the type of drug and the purpose and method of treatment. A drug injection device using a drug pump is capable of automatically injecting a drug into a body at a desired speed and dosage at a required time. Accordingly, the drug injection device using a drug pump may be utilized in various forms as well as being used in hospitals or in a patient's daily living environment.

Generally, an insulin pump, which is commonly referred to as an insulin injector, is a medical device for diabetes patients in whom insulin is not secreted or is secreted only in a small amount, and performs a role similar to that of a pancreas by supplying insulin from outside the body into the body at predetermined times to control blood glucose.

Such an insulin pump is used by insulin-dependent diabetes patients, and is capable of continuously injecting a drug for 24 hours while being attached to a patient. As described above, since the insulin injector needs to periodically inject a drug into a diabetes patient for a long period of time, development of technologies for miniaturization and automation of the insulin injector has been actively conducted for user convenience.

The insulin injector is initially in a state in which a drug is not charged in a drug storage unit, and operation of the insulin injector is started as the charging of the drug in the drug storage unit is detected.

Therefore, there is a need for a device capable of effectively detecting charging of a drug in the drug storage unit.

### SUMMARY

### [Problem to be Solved]

The present invention has been made to solve the above-described problems, and an object of the present invention is to provide a drug injection device including a drug injection detection unit that detects a thickness change of a drug storage unit.

However, the technical problem to be achieved by the present embodiment is not limited to the above-described technical problem, and other technical problems may exist.

### [Means for Solving the Problem]

As a technical means for solving the above-described technical problem, a drug injection detection unit according to an embodiment of the present invention comprises: a support portion spaced apart from one side surface of a drug storage unit by a predetermined distance; a sensor coupled to one side of the support portion and disposed to be in contact with the one side surface of the drug storage unit, the sensor being configured to detect drug injection based on a thickness change of the drug storage unit; and a switch that operates according to a state of the sensor to be turned on and outputs a control signal upon being turned on.

In addition, a drug injection device according to an embodiment of the present invention comprises: a drug storage unit having a thickness that changes according to an amount of a drug injected therein; a drug injection detection unit configured to detect a thickness change of the drug storage unit and output a control signal for initiating operation of the drug injection device; and a controller configured to initiate operation of the drug injection device based on the control signal.

In addition, an operating method of a drug injection device according to an embodiment of the present invention comprises: injecting a drug into a drug storage unit of the drug injection device; detecting drug injection, by a drug injection detection unit disposed to be in contact with the drug storage unit, based on a thickness change of the drug storage unit; and outputting, when the drug injection is detected, by the drug injection detection unit, a control signal for initiating operation of the drug injection device to a controller of the drug injection device.

### [Effects of the Invention]

According to the means for solving the problem of the present invention described above, a rotation angle of a sensor arm varies according to a thickness change of the drug storage unit, thereby enabling drug injection into the drug storage unit to be easily detected.

### [Brief Description of the Drawings]

FIG. 1 is a front perspective view of a drug injection device according to an embodiment of the present invention.
FIG. 2 is a rear perspective view of the drug injection device according to an embodiment of the present invention.
FIG. 3 is a plan view of an interior of a housing shown in FIG. 1.
FIG. 4 is a plan view of the drug injection device from which a cover portion shown in FIG. 1 is removed.
FIG. 5 is a plan view of the drug injection device from which a contact surface shown in FIG. 2 is removed.
FIG. 6 is a plan view of a drug storage unit shown in FIG. 4.
FIG. 7 is a perspective view of a pump assembly shown in FIG. 4.
FIG. 8 is a cross-sectional view of a port connector shown in FIG. 7 taken in a first direction.
FIG. 9 is a cross-sectional view of the port connector shown in FIG. 7 taken in a second direction.
FIG. 10 is a block diagram schematically illustrating a configuration of an electroosmotic pump shown in FIG. 7.
FIG. 11 is a conceptual diagram schematically illustrating a configuration of a driving unit shown in FIG. 10.
FIG. 12 is an exemplary diagram schematically illustrating an operation of the driving unit shown in FIG. 11.
FIG. 13 is a perspective view illustrating a coupling structure between the drug injection detection unit and the drug storage unit shown in FIG. 5.
FIG. 14 is a perspective view of the drug injection detection unit shown in FIG. 13.
FIGS. 15 and 16 are exemplary diagrams illustrating operations of a sensor shown in FIG. 14.
FIG. 17 is a flowchart illustrating an operating method of a drug injection device.

### [Detailed Description]

Hereinafter, the present invention will be described in detail with reference to the accompanying drawings. However, the present invention may be implemented in various different forms and is not limited to the embodiments described herein. In addition, the accompanying drawings are provided only to facilitate understanding of the embodiments disclosed in the present specification, and the technical spirit disclosed in the present specification is not limited by the accompanying drawings. In order to clearly describe the present invention in the drawings, parts irrelevant to the description are omitted, and the sizes, shapes, and forms of respective components illustrated in the drawings may be variously modified. Throughout the specification, the same or similar reference numerals are assigned to the same or similar parts.

Suffixes such as "assembly" and "unit" used for components in the following description are assigned or used interchangeably only for convenience of description, and do not have meanings or roles that distinguish them from each other per se. In addition, in describing the embodiments disclosed in the present specification, detailed descriptions of related known technologies are omitted when it is determined that such descriptions may obscure the gist of the embodiments disclosed in the present specification.

Throughout the specification, when a part is described as being "connected (coupled, contacted, or joined)" to another part, this includes not only cases in which the part is "directly connected (coupled, contacted, or joined)" to the other part, but also cases in which the part is "indirectly connected (coupled, contacted, or joined)" to the other part with another member interposed therebetween. In addition, when a part is described as "including (comprising or being provided with)" a component, this means that other components may be further included (comprised or provided), unless otherwise specifically stated, rather than excluding other components.

Terms indicating an ordinal number such as first, second, and the like, as used in the present specification, are used only for the purpose of distinguishing one component from another component, and do not limit an order or relationship of the components. For example, a first component of the present invention may be referred to as a second component, and similarly, a second component may also be referred to as a first component.

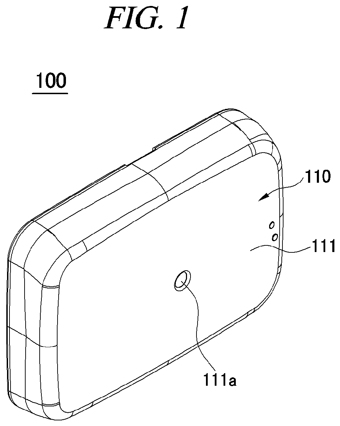

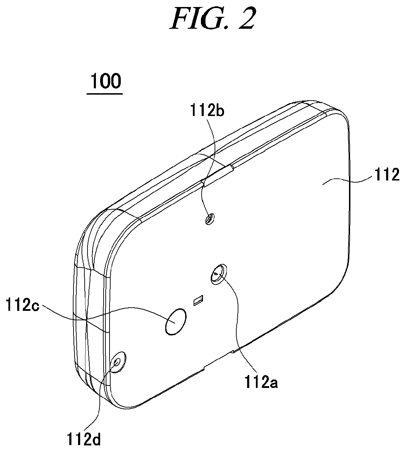

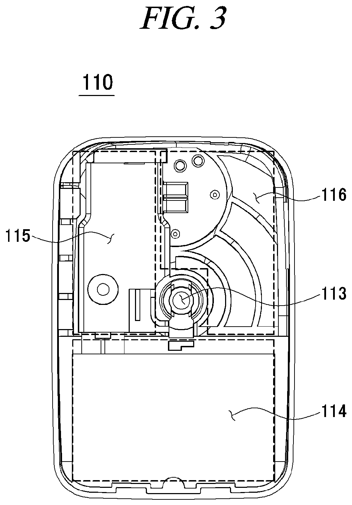

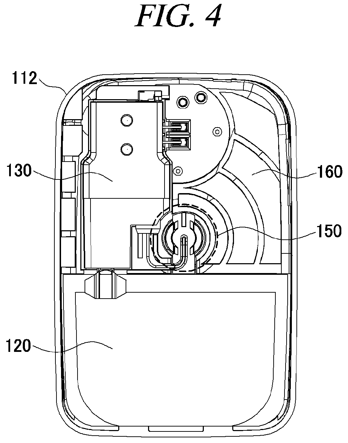

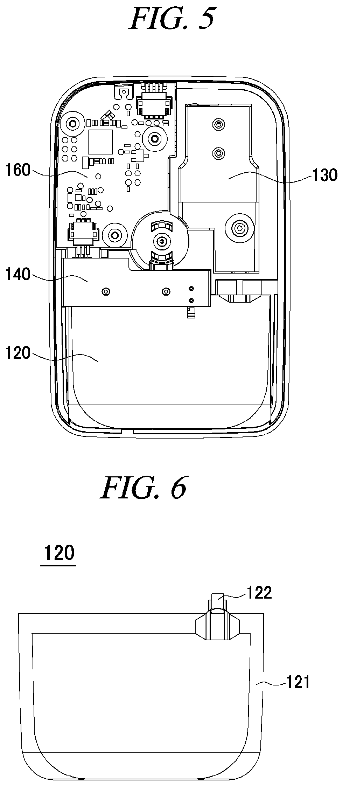

FIG. 1 is a front perspective view of a drug injection device according to an embodiment of the present invention, FIG. 2 is a rear perspective view of the drug injection device according to an embodiment of the present invention, FIG. 3 is a plan view of an interior of a housing shown in FIG. 1, FIG. 4 is a plan view of the drug injection device from which a cover portion shown in FIG. 1 is removed, and FIG. 5 is a plan view of the drug injection device from which a contact surface shown in FIG. 2 is removed.

Referring to FIGS. 1 to 5, a drug injection device (100) according to an embodiment of the present invention comprises a housing (110), and a drug storage unit (120), a pump assembly (130), a drug injection detection unit (140), an injection assembly (150), and a controller (160) disposed inside the housing (110).

Referring to FIGS. 1 to 5, the housing (110) may include a cover portion (111) and a contact surface (112), and a mounting space is formed between the cover portion (111) and the contact surface (112).

The cover portion (111) is formed with rounded edges so as to allow the patch-type drug injection device (100) to remain attached. The contact surface (112) is a component attached to a user's body, and is formed with a discharge port (112a) for drawing an insertion tube to the outside. Here, a silicone cap may be coupled to the discharge port (112a), and the insertion tube inserted into the user may be fixed by the silicone cap.

The mounting space is divided into a central region (113) and first to third regions (114, 115, 116), and the first to third regions (114, 115, 116) are formed to surround the central region (113). In particular, the mounting space in the housing (110) is divided into the central region (113) and the first to third regions (114, 115, 116) extending in a direction perpendicular to an insertion direction of an insertion tube. Major components are coupled to each region. In general, a PCB controller is first disposed over an entire plane or most of the plane of a device, and a drug storage unit or a pump is disposed thereon, which causes a problem in that a thickness of the device is increased. However, in order to reduce the thickness, the patch-type drug injection device (100) according to the present invention minimizes overlapping regions of the respective components.

Referring to FIGS. 4 and 5, the drug storage unit (120), the pump assembly (130), the drug injection detection unit (140), the injection assembly (150), and the controller (160) may be disposed in the central region (113) and the first to third regions (114, 115, 116). The drug storage unit (120) and the drug injection detection unit (140) may be disposed in the first region (114), the pump assembly (130) may be disposed in the second region (115), the controller (160) may be disposed in the third region (116), and the injection assembly (150) may be disposed in the central region (113). Accordingly, the drug storage unit (120), the pump assembly (130), the drug injection detection unit (140), and the controller (160) may be disposed to surround the injection assembly (150). Here, the drug storage unit (120) and the drug injection detection unit (140) may be disposed side by side in the first region (114).

As described above, the injection assembly (150) including an insertion tube is disposed in the central region (113), such that the insertion tube is prevented from being detached and remains injected into a user even when the user moves or an external impact is applied.

Next, the drug storage unit (120), the pump assembly (130), the drug injection detection unit (140), the injection assembly (150), and the controller (160) will be described in detail.

FIG. 6 is a plan view of the drug storage unit shown in FIG. 4. Referring to FIG. 6, the drug storage unit (120) stores a drug to be injected, and has a thickness that changes according to an amount of the drug injected. The drug storage unit (120) may be provided as a flat pouch bag made of a polymer material. A flange portion (121) may be formed around a periphery of the drug storage unit (120), in which two surfaces constituting the drug storage unit (120) are bonded to each other by a predetermined width. The flange portion (121) may limit expansion of the drug storage unit (120) toward a peripheral side thereof.

The drug storage unit (120) is formed of a release paper, and an interior of the drug storage unit (120) and an exterior of the drug storage unit (120) are respectively made of materials having different melting temperatures, wherein a melting temperature of the interior material is lower than a melting temperature of the exterior material. The drug storage unit (120) is manufactured by applying heat at a predetermined temperature at which the interior material melts to form the flange portion (121). When heat is applied to a side surface, the interior materials adhere to each other to be sealed, and a lower portion is folded into a W shape and heated. At this time, the interior materials adhere to each other while the exterior materials do not adhere to each other, thereby forming a wrinkled shape to form the flange portion (121).

In addition, a drug inlet and outlet (122) through which a drug enters and exits is coupled to the drug storage unit (120), such that a drug is introduced into the drug storage unit (120) or a drug stored in the drug storage unit (120) is discharged to the outside.

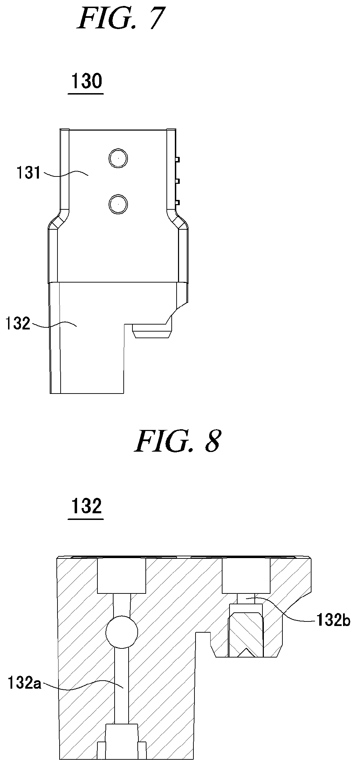

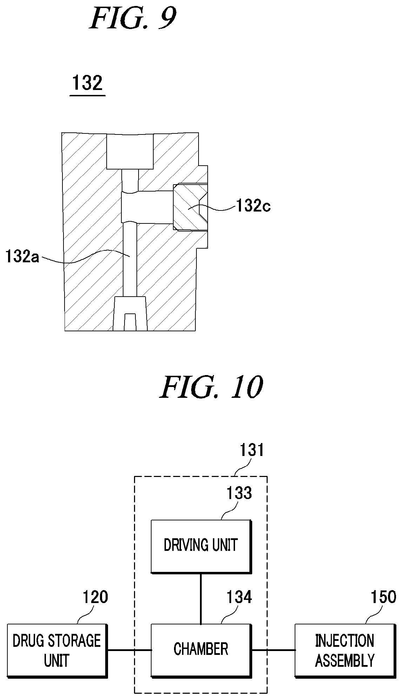

FIG. 7 is a perspective view of the pump assembly shown in FIG. 4, FIG. 8 is a cross-sectional view of a port connector shown in FIG. 7 taken in a first direction, and FIG. 9 is a cross-sectional view of the port connector shown in FIG. 7 taken in a second direction.

Hereinafter, the pump assembly (130) will be described with reference to FIGS. 7 to 9. The pump assembly (130) is connected to the drug storage unit (120), and draws in a drug from the drug storage unit (120) and discharges the drug to an insertion tube of the injection assembly (150). The pump assembly (130) may include an electroosmotic pump (131) and a port connector (132). The electroosmotic pump (131) is an electrochemically driven pump, which will be described in detail later.

Referring to FIG. 8, the port connector (132) is formed therein with an inflow passage (132a) through which a drug flows from the drug storage unit (120) to the electroosmotic pump (131), and an outflow passage (132b) through which the drug is discharged from the electroosmotic pump (131) to the injection assembly (150). In addition, referring to FIG. 9, the port connector (132) may further include a drug injection passage (132c). The drug injection passage (132c) is formed in a direction intersecting a traveling direction of the first passage (132a), has one end communicating with the first passage (132a), and has the other end open to the outside. The drug injection passage (132c) is a passage through which a drug may be injected into the first passage (132a) using a drug injection syringe, and is used when a drug is charged into the drug storage unit (120) at an initial stage of operation of the drug injection device (100) according to the present invention.

Next, the electroosmotic pump (131) will be described in detail with reference to FIGS. 10 to 12.

FIG. 10 is a block diagram schematically illustrating a configuration of the electroosmotic pump (131) shown in FIG. 7.

Referring to FIG. 10, the electroosmotic pump (131) will be described in detail. The electroosmotic pump (131) includes a driving unit (133) and a chamber (134). The driving unit (133) is electrochemically driven by a control signal to generate positive pressure and negative pressure, and discharges a drug according to the positive pressure and the negative pressure. The chamber (134) draws in a drug from the drug storage unit (120) according to pressure generated by the driving unit (133), and then discharges the drug to the injection assembly (150). Here, the drug is a drug to be injected into a specific patient, and insulin injected into a diabetes patient may be taken as an example.

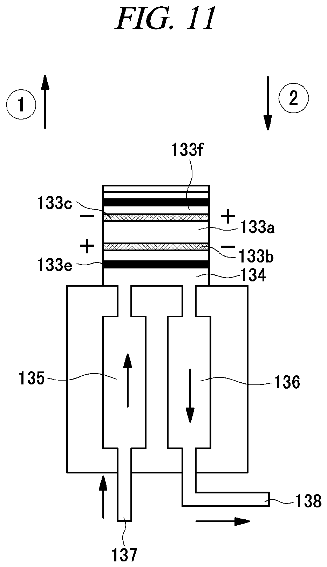

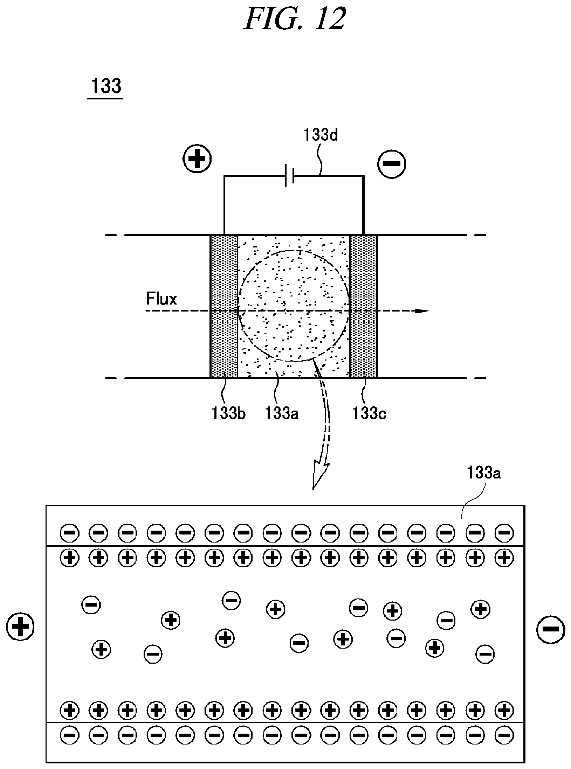

FIG. 11 is a conceptual diagram schematically illustrating a configuration of the driving unit shown in FIG. 10, and FIG. 12 is an exemplary diagram schematically illustrating the configuration of the driving unit shown in FIG. 11.

Referring to FIGS. 11 and 12, the driving unit (133) will be described in detail. The driving unit (133) is a pump that utilizes movement of a fluid caused by an electroosmotic phenomenon generated when a voltage or a current is applied to both ends of a pumping portion (membrane, 133a) using electrodes. The driving unit (133) may include a membrane (133a), a first electrode (133b) and a second electrode (133c) disposed on opposite sides of the membrane (133a), a power source (133d) configured to apply a voltage or a current to the first electrode (133b) and the second electrode (133c), and a first diaphragm (133e) and a second diaphragm (133f) for movement of a fluid. Each of the diaphragms (133e, 133f) is provided on one side and the other side of the pumping portion (133a), and is deformed by movement of a pumping fluid as positive pressure and negative pressure are alternately generated. For example, the first diaphragm (133e) and the second diaphragm (133f) transmit the negative pressure and the positive pressure generated by driving of the pumping portion (133a) to a target fluid. More specifically, when negative pressure is generated, at least a portion of the first diaphragm (133e) and the second diaphragm (133f) moves backward (moves in a direction indicated by ①), and the target fluid is sucked into the chamber (134). Conversely, when positive pressure is generated, at least a portion of the first diaphragm (133e) and the second diaphragm (133f) moves forward (moves in a direction indicated by ②), and the target fluid is discharged from the chamber (134).

As a material of the membrane (133a), silica, glass, or the like is generally used, and such materials exhibit a negative surface charge when immersed in an aqueous solution. The membrane (133a) has numerous passages through which a fluid may pass, and when one of the passages is enlarged, a surface of a fluid passage having a negative charge (bound anion) may be charge-balanced by mobile cations having a positive charge. In this state, when a positive voltage is applied to the first electrode (133b) and a negative voltage is applied to the second electrode (133c), negative pressure is generated, and due to such negative pressure, a fluid inside the driving unit (133) moves in the direction indicated by ①. At this time, a drug is drawn in through a suction passage (136), passes through a suction valve (135), and flows into the chamber (134). At this time, a discharge valve (137) is closed so that negative pressure is not transmitted to a discharge passage (138). Conversely, when a negative voltage is applied to the first electrode (133b) and a positive voltage is applied to the second electrode (133c), positive pressure in an opposite direction is generated by a reversible electrochemical reaction. Due to such positive pressure, the fluid inside the driving unit (133) moves in the direction indicated by ②. At this time, the drug stored in the chamber (134) is injected into a target object through the discharge passage (138) via the discharge valve (137). At this time, the suction valve (135) is blocked so that positive pressure is not transmitted to the suction passage (136).

Such a phenomenon is referred to as an electroosmotic phenomenon, and a pump utilizing this principle is the electroosmotic pump (131). In the present invention, since the electroosmotic pump (131) is used as a driving means in the patch-type drug injection device (100), the driving means may be manufactured to be thinner compared to other technologies using an electric motor or the like. Accordingly, an overall thickness of the patch-type drug injection device (100) may be reduced, thereby enabling more stable attachment to skin.

Referring back to FIG. 5, the drug injection detection unit (140) detects a thickness change of the drug storage unit (120) and controls operation of the drug injection device (100) according to a change in a stored amount of a drug.

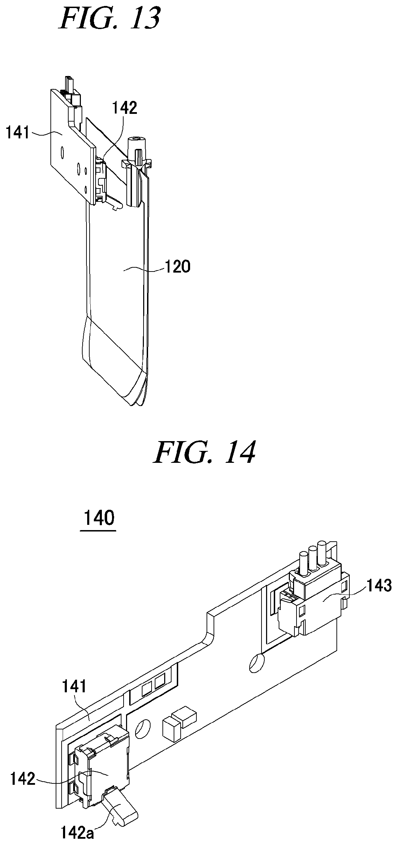

Next, a detailed configuration of the drug injection detection unit (140) will be described. FIG. 13 is a perspective view illustrating a coupling structure between the drug injection detection unit and the drug storage unit shown in FIG. 5, FIG. 14 is a perspective view of the drug injection detection unit shown in FIG. 13, and FIGS. 15 and 16 are exemplary diagrams illustrating operations of a sensor shown in FIG. 14.

Referring to FIG. 13, the drug injection detection unit (140) is disposed on one side of the drug storage unit (120), and detects a thickness change of the drug storage unit (120) into which a drug is injected, thereby controlling operation of the drug injection device (100) according to a change in an amount of drug stored in the drug storage unit (120). When the drug injection device (100) is initially used, the drug injection detection unit (140) detects injection of a drug into the drug storage unit (120) and operates the drug injection device (100).

Specifically, referring to FIG. 14, the drug injection detection unit (140) may include a support portion (141), a sensor (142), and a switch (not shown). The support portion (141) is disposed in a mounting space of the drug injection device (100) while being spaced apart from one side surface of the drug storage unit (120) by a predetermined distance, and the support portion (141) may be implemented as a PCB.

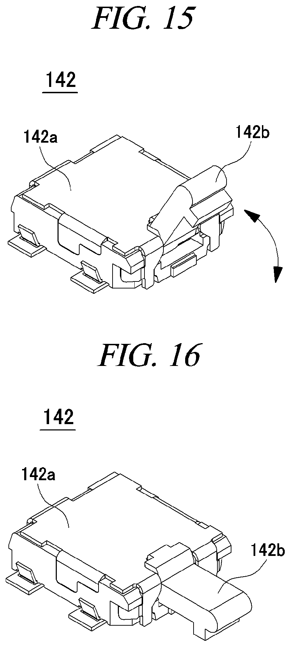

Referring to FIGS. 15 and 16, the sensor (142) is coupled to one side of the support portion (141) and is disposed to be in contact with one side surface of the drug storage unit (120), thereby detecting injection of a drug into the drug storage unit (120) based on a thickness change of the drug storage unit (120). The sensor (142) may include a body (142a) coupled to the support portion (141) and a sensor arm (142b) that pivots along a pivot axis. An end of the sensor arm (142b) may be disposed to be in contact with one side surface of the drug storage unit (120). Specifically describing operation of the sensor (142), when a drug is injected into the drug storage unit (120) and a thickness of the drug storage unit (120) increases, the drug storage unit (120) presses the sensor arm (142b), thereby detecting drug injection.

The sensor arm (142b) may initially be in a state pivoted to form a predetermined angle with the body (142a) of the sensor (142), as shown in FIG. 15. In this state, an end of the sensor arm (142b) is in contact with one surface of the drug storage unit (120). Thereafter, when a drug is injected into the drug storage unit (120), the drug storage unit (120) expands, and as the drug storage unit (120) expands, the sensor arm (142b) is pressed. Accordingly, as a volume of the drug storage unit (120) increases, the sensor arm (142b) is pressed and a pivot angle of the sensor arm (142b) changes as shown in FIG. 16, during which the switch (not shown) is operated. As a result, a control signal for initiating operation of the drug injection device may be output to a controller of the drug injection device (100).

Accordingly, since the sensor arm (142b) is pressed and pivoted due to an increase in thickness of the drug storage unit (120), it is possible to detect that drug charging has been initiated. However, the sensor (142) is not limited to a configuration including the sensor arm (142b), and any sensor capable of detecting a thickness change of the drug storage unit (120) may be applied.

The switch (not shown) is turned on according to a state of the sensor (142), and outputs a control signal for controlling operation of the drug injection device (100) when turned on. The switch (not shown) may be embedded in the body (142a) of the sensor (142), and may be turned on according to a pivot angle of the sensor arm (142b) to output a control signal for controlling operation of the drug injection device (100) to the controller. Specifically, when the pivot angle of the sensor arm (142b) is equal to or less than a preset angle, the switch (not shown) is turned on and outputs, to the controller (160), a control signal for initiating operation of the drug injection device (100). That is, assuming that FIG. 15 illustrates an initial state in which the sensor arm (142b) and an extending direction of the body (142a) of the sensor (142) or the support portion (141) form a first angle, the switch (not shown) is turned on when, due to pivoting of the sensor arm (142b), a second angle smaller than the first angle is reached, or when an angle of 0 degrees is formed as shown in FIG. 16.

In addition, the drug injection detection unit (140) may further include a communication unit (143) configured to transmit a control signal to the controller of the drug injection device (100).

Referring back to FIG. 4, the injection assembly (150) includes an insertion tube and injects a drug discharged from the pump assembly (130) into an injection target, and the controller (160) has electronic components mounted thereon that are required for driving and controlling the pump assembly (130), and includes a control unit configured to operate the drug injection device (100) through a control signal transmitted from the drug injection detection unit (140).

Next, additional configurations of the patch-type drug injection device (100) will be described with reference to FIGS. 1 and 2.

Referring to FIG. 1, the cover portion (111) of the housing (110) may be formed with a shooting hole (111a) in a region corresponding to a region in which the injection assembly (150) is disposed. The shooting hole (111a) is a hole through which an external force is transmitted to the injection assembly (150) by an applicator (not shown) so as to insert the insertion tube into a user. A silicone cap may be coupled to the shooting hole (111a).

Referring to FIG. 2, the contact surface (112) of the housing (110) may be formed with a drug injection hole (112b) and an air vent hole (112c). The drug injection hole (112b) is a hole for supplying, from the outside, a drug to be stored in the drug storage unit (120), and the air vent hole (112c) is a hole for eliminating a pressure difference between the inside and the outside of the housing (110).

In addition, the patch-type drug injection device (100) may include a sound output device (not shown) inside the housing (110) to provide a notification for a specific situation. The controller (160) may include a switch for stopping operation of the sound output device (not shown), and the contact surface (112) may be formed with a buzzer stop hole (112d) that provides a passage for operating the switch of the controller (160). Here, a silicone cap may be coupled to the drug injection hole (112b), and an air vent sticker may be attached to the air vent hole (112c) and the buzzer stop hole (112d).

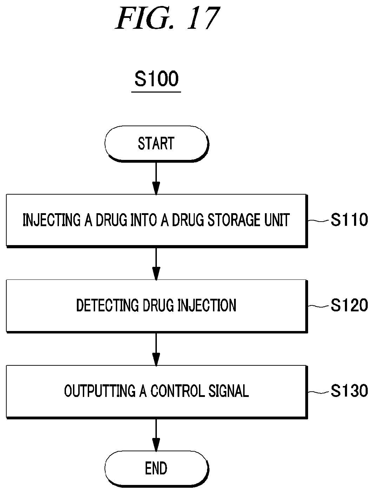

FIG. 17 is a flowchart for explaining an operating method of a drug injection device.

Referring to FIGS. 2, 13, 14, and 17, an operating method (S100) of the drug injection device will be described. In an initial operating method (S100) of the drug injection device, when a drug is injected into the drug storage unit (120) through the drug injection hole (112b) by a user (step S110), the drug injection detection unit (140) detects drug injection based on a thickness change of the drug storage unit (120) (step S120). When drug injection is detected in step S120, the drug injection detection unit (140) outputs, to the controller of the drug injection device (100), a control signal for initiating operation of the drug injection device (100) (step S130). Then, the drug injection device (100) is operated.

In step S120, drug injection is detected when the sensor arm (142b) is pressed due to a thickness change of the drug storage unit (120) and a pivot angle of the sensor arm (142b) becomes equal to or less than a preset angle. When drug injection is detected, in step S130, a switch of the sensor (142) outputs a control signal to the controller (160) of the drug injection device (100).

Those skilled in the art to which the present invention pertains will understand that various modifications and variations may be made without departing from the technical spirit or essential features of the present invention based on the above description. Therefore, the embodiments described above should be understood as being illustrative in all respects and not restrictive. The scope of the present invention is defined by the claims described below, and all changes or modifications derived from the meaning and scope of the claims and equivalents thereof should be interpreted as being included in the scope of the present invention.

The scope of the present application is defined by the claims described below rather than the detailed description set forth above, and all changes or modifications derived from the meaning and scope of the claims and equivalents thereof should be interpreted as being included in the scope of the present application.

## Claims

1. A drug injection detection unit configured to detect injection of a drug into a drug storage unit, comprising:
a support portion spaced apart from one side surface of the drug storage unit by a predetermined distance;
a sensor coupled to one side of the support portion and disposed to be in contact with the one side surface of the drug storage unit, the sensor being configured to detect drug injection based on a thickness change of the drug storage unit; and
a switch configured to operate according to a state of the sensor to be turned on and to output a control signal upon being turned on,
wherein the sensor comprises:
a body coupled to the support portion; and
a sensor arm coupled to a pivot of the body and configured to pivot along a pivot axis,
and wherein an end of the sensor arm is disposed to be in contact with the one side surface of the drug storage unit.

2. The drug injection detection unit of claim 1,
wherein the switch is initially in a turned-off state,
and outputs, to a controller of a drug injection device, a control signal for initiating operation of the drug injection device when the switch is turned on by the sensor as a pivot angle of the sensor arm becomes equal to or less than a preset angle.

3. The drug injection detection unit of claim 2,
wherein the switch is embedded in the body of the sensor and is turned on by pivoting of the sensor arm.

4. A drug injection device comprising:
a drug storage unit having a thickness that changes according to an amount of a drug injected;
a drug injection detection unit configured to detect a thickness change of the drug storage unit and output a control signal for initiating operation of the drug injection device;
a controller configured to initiate operation of the drug injection device based on the control signal; and
a pump assembly connected to the drug storage unit and configured to draw in and discharge a drug stored in the drug storage unit,
wherein a drug is injected into the drug storage unit through a drug injection passage formed at one side of the pump assembly,
and wherein the drug injection detection unit comprises:
a support portion spaced apart from one side surface of the drug storage unit by a predetermined distance;
a sensor coupled to one side of the support portion and disposed to be in contact with the one side surface of the drug storage unit, the sensor being configured to detect drug injection based on a thickness change of the drug storage unit; and
a switch configured to be turned on according to a state of the sensor and to output a control signal upon being turned on.

5. The drug injection device of claim 4,
wherein the sensor comprises:
a body coupled to the support portion; and
a sensor arm coupled to a pivot of the body and configured to pivot along a pivot axis,
wherein an end of the sensor arm is disposed to be in contact with the one side surface of the drug storage unit.

6. The drug injection device of claim 4,
wherein the switch is initially in a turned-off state,
and outputs, to the controller,
a control signal for initiating operation of the drug injection device when the switch is turned on by the sensor as a pivot angle of the sensor arm becomes equal to or less than a preset angle.

7. The drug injection device of claim 6,
wherein the switch is embedded in the body of the sensor and is turned on by pivoting of the sensor arm.

8. The drug injection device of claim 4,
wherein the drug storage unit has a pouch structure in which two surfaces are bonded to each other, and as the drug is charged, the thickness increases such that one side surface presses the other end of the sensor arm.

9. An operating method of a drug injection device, comprising:
injecting a drug into a drug storage unit of the drug injection device;
detecting drug injection, by a drug injection detection unit disposed to be in contact with the drug storage unit, based on a thickness change of the drug storage unit; and
outputting, when the drug injection is detected, by the drug injection detection unit, a control signal for initiating operation of the drug injection device to a controller of the drug injection device,
wherein the detecting of the drug injection comprises detecting drug injection when a sensor arm is pressed due to a thickness change of the drug storage unit and a pivot angle of the sensor arm becomes equal to or less than a preset angle.
